# EUROPEAN PATENT APPLICATION

(11) **EP 2 781 915 A1**
(43) Date of publication of application: **24.09.2014**
(21) Application number: 12849902.7
(22) Date of filing: 10.07.2012
(51) Int. Cl.: G01N 27/416, G01N 27/327

(54) **METHOD FOR MEASURING SUBSTANCES**

(30) Priority: 18.11.2011 JP 2011252765
(71) Applicant: Murata Manufacturing Co., Ltd., Kyoto 617-8555 (JP)
(72) Inventor: OOE, Hideaki, Nagaokakyo-shi Kyoto 617-8555 (JP); TAKAGI, Jun, Nagaokakyo-shi Kyoto 617-8555 (JP); YOKOYAMA, Kenji, Tokyo 100-8921 (JP); HIRATSUKA, Atsunori, Tokyo 100-8921 (JP); YOSHIDA, Nobuyuki, Tokyo 100-8921 (JP); SASAKI, Noriko, Tokyo 100-8921 (JP)
(74) Representative: Behr, Wolfgang
(86) International application number: PCT/JP2012/004442
(87) International publication number: WO 2013/073074

(57) **Abstract**

A technique allowing improvement in determination accuracy in quantification of a substance to be determined, which is contained in a specimen, by lessening influence by a current component different from an oxidation current resulting from oxidation of a reducing substance generated through reaction between an enzyme and the substance to be determined in the specimen, of current components contained in a response current resulting from application of a determination potential referenced to a counter electrode to a working electrode, is provided. Since a conditioning potential higher than a determination potential is applied as a pulse to the working electrode, influence by a current component different from an oxidation current resulting from oxidation of a reducing substance generated through reaction between an enzyme and a substance to be determined in the specimen, of current components contained in a response current resulting from application of the determination potential referenced to the counter electrode to the working electrode, can be lessened. Thus, the response current can be measured in a stable manner and determination accuracy in quantification of a substance to be determined which is contained in a specimen can be improved.

## Description

### TECHNICAL FIELD

The present invention relates to a substance determination method for quantification of a substance to be determined, which is contained in a specimen, with the use of a biosensor.

### BACKGROUND ART

A substance determination method for quantification of a substance to be determined by measuring, by using a biosensor having a cavity into which a specimen is supplied; an electrode system including a working electrode and a counter electrode; and a reaction layer containing an enzyme reacting specifically with the substance to be determined, an oxidation current resulting from oxidation, through application of a determination potential referenced to the counter electrode to the working electrode, of a reducing substance generated through reaction between the substance to be determined, which is contained in the specimen supplied into the cavity, and the reaction layer has conventionally been known.

A biosensor is formed by stacking an electrode layer obtained by providing an electrode on an insulating substrate composed of polyethylene terephthalate, a cover layer, and a spacer layer arranged between the electrode layer and the cover layer. A slit for forming a cavity into which a specimen is to be supplied is provided in the spacer layer. As the cover layer is stacked on and bonded to the electrode layer with the spacer layer being interposed, the cavity into which a specimen such as a blood sample is to be supplied is formed by the electrode layer and the cover layer and by a slit portion in the spacer layer, and a specimen introduction port is formed in a side surface of the biosensor. When the specimen is supplied into the cavity through the specimen introduction port, the specimen is supplied into the cavity owing to a capillary phenomenon. Therefore, an air vent communicating with a terminal portion of the formed cavity is formed in the cover layer.

As the working electrode and the counter electrode as well as an electrode pattern electrically connected to the working electrode and the counter electrode are provided in the electrode layer, the electrode system is formed on the electrode layer. The working electrode and the counter electrode are provided on the electrode layer such that they are partially exposed at the cavity formed in the biosensor, and the reaction layer is provided on a part of the working electrode and the counter electrode exposed at the cavity. Therefore, as a specimen is supplied into the cavity through the specimen introduction port, the specimen comes in contact with each electrode and the reaction layer exposed at the cavity, and the reaction layer is dissolved in the specimen.

The reaction layer provided on the working electrode and the counter electrode contains, for example, glucose oxidase reacting specifically with glucose contained in the specimen and potassium ferricyanide serving as a mediator (electron acceptor). Then, ferricyanide ions resulting from solution of potassium ferricyanide in the specimen are reduced to ferrocyanide ions representing a reductant by electrons emitted at the time of reaction of glucose with glucose oxidase and following oxidation into gluconolactone. Therefore, as the specimen containing glucose is supplied through the specimen introduction port into the cavity formed in the biosensor, ferricyanide ions are reduced by electrons emitted as a result of oxidation of glucose, and hence ferrocyanide ions representing a reductant of ferricyanide ions are generated in an amount in accordance with a concentration of glucose contained in the specimen and oxidized through enzyme reaction.

According to the biosensor as such, magnitude of an oxidation current resulting from oxidation of a reductant of a mediator resulting from enzyme reaction on the working electrode is dependent on a concentration of glucose in the specimen. Therefore, glucose contained in the specimen can be quantified by measuring this oxidation current.

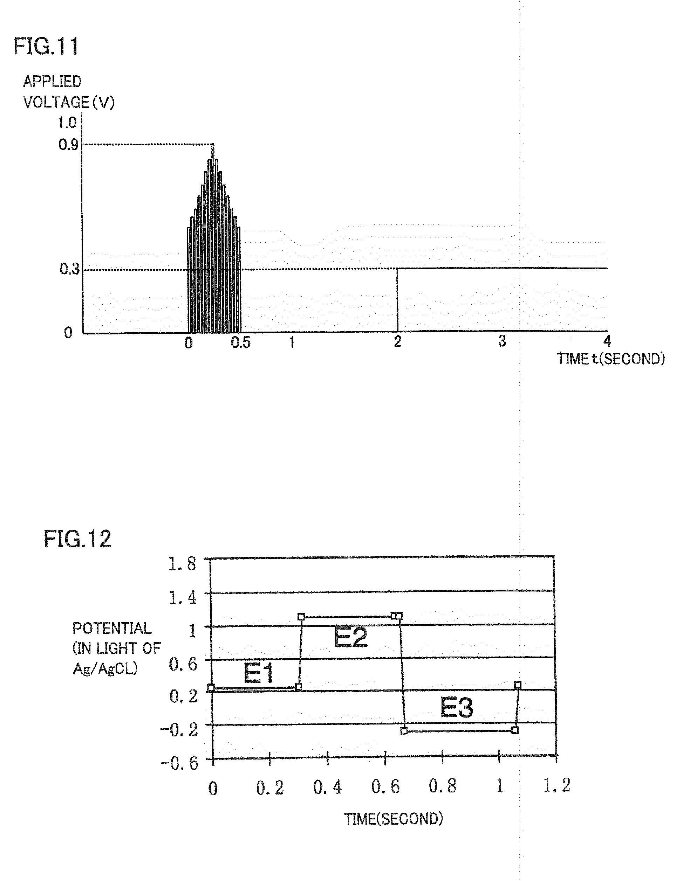

In order to improve accuracy in determination of an oxidation current, for the purpose of removal of such an impurity as soil or dust adhering to a working electrode and a counter electrode exposed at a cavity in a biosensor, a conditioning potential is applied to the working electrode with the counter electrode being defined as the reference before a determination potential referenced to the counter electrode is applied to the working electrode. For example, in the substance determination method described in PTD 1, as shown in the diagram for illustrating one example of the conventional substance determination method in Fig. 12, a conditioning potential E1 lower than a determination potential E2 is applied to the working electrode before determination potential E2 referenced to the counter electrode is applied to the working electrode.

Therefore, as conditioning potential E1 is applied to the working electrode prior to measurement of an oxidation current, impurities adhering to the working electrode and the counter electrode are removed as a result of electrochemical reaction. Therefore, a current component resulting from electrochemical reaction of impurities adhering to the working electrode and the counter electrode, of current components contained in a response current resulting from application of a determination potential referenced to the counter electrode to the working electrode, can be decreased, and a ratio of the oxidation current contained in the response current can be increased. Thus, improvement in accuracy in measurement of an oxidation current to be measured can be expected.

### CITATION LIST

### PATENT DOCUMENT

PTD 1: Japanese National Patent Publication No. 2009-533690 (paragraphs 0008 to 0012 and Abstract)

### SUMMARY OF INVENTION

### TECHNICAL PROBLEM

With the conventional substance measurement method described above, a conditioning potential lower than a determination potential is applied to a working electrode. Therefore, an impurity which electrochemically reacts at the time of application of a determination potential higher than a conditioning potential to a working electrode cannot be removed from the working electrode and a counter electrode, and hence improvement in the technique has been demanded.

The present invention was made in view of the problem above, and an object thereof is to provide a technique allowing improvement in determination accuracy in quantification of a substance to be determined which is contained in a specimen, by lessening influence by a current component different from an oxidation current resulting from oxidation of a reducing substance generated through reaction between the substance to be determined in the specimen and an enzyme, of current components contained in a response current resulting from application of a determination potential referenced to a counter electrode to a working electrode.

### SOLUTION TO PROBLEM

In order to achieve the object above, a substance determination method according to the present invention is a substance determination method for quantification of a substance to be determined by measuring, by using a biosensor having a cavity into which a specimen is supplied; an electrode system including a working electrode and a counter electrode; and a reaction layer containing an enzyme reacting specifically with a substance to be determined, an oxidation current resulting from oxidation, through application of a determination potential referenced to the counter electrode to the working electrode, of a reducing substance generated through reaction between the substance to be determined which is contained in the specimen supplied into the cavity and the reaction layer, the substance determination method including applying to the working electrode at least once, a pulse of conditioning potential higher than the determination potential with the counter electrode being defined as a reference after the specimen is supplied into the cavity and before the determination potential is applied to the working electrode.

The determination potential is equal to or higher than an oxidation potential at which the reducing substance is oxidized (claim 2).

The conditioning potential is lower in potential than a decomposition voltage of water (claim 3).

A pulse width in application of the conditioning potential to the working electrode is from 30 to 750 milliseconds (claim 4).

The conditioning potential is applied before lapse of one second since sensing of supply of the specimen into the cavity (claim 5).

The determination potential is applied after lapse of at least one second since sensing of supply of the specimen into the cavity (claim 6).

The cavity has a volume smaller than 0.6 µl (claim 7).

### ADVANTAGEOUS EFFECTS OF INVENTION

According to the invention in claim 1, after a specimen is supplied into a cavity in a biosensor and before a determination potential for measuring an oxidation current resulting from oxidation of a reducing substance generated through reaction between a substance to be determined in the specimen and an enzyme is applied to a working electrode, a pulse of conditioning potential higher than a determination potential with a counter electrode being defined as the reference is applied to the working electrode at least once. Therefore, an impurity adhering to the working electrode and the counter electrode which electrochemically reacts at the time of application of the determination potential to the working electrode electrochemically reacts as a conditioning potential is applied to the working electrode with the counter electrode being defined as the reference, and is removed from the working electrode and the counter electrode. Therefore, influence by a current component different from an oxidation current resulting from oxidation of a reducing substance generated through reaction between the substance to be determined in the specimen and the enzyme, of current components contained in a response current resulting from application of a determination potential referenced to the counter electrode to the working electrode, can be lessened. Thus, determination accuracy in quantification of the substance to be determined which is contained in the specimen can be improved.

According to the invention in claim 2, since the determination potential is equal to or higher than the oxidation potential at which the reducing substance resulting from enzyme reaction of the substance to be determined is oxidized, variation in concentration of the reducing substance in the specimen can be suppressed without increase in an amount of reducing substance contained in the specimen due to reduction reaction caused by application of the determination potential to the working electrode, and an oxidation current resulting from oxidation of the reducing substance can be stabilized. Therefore, determination accuracy in quantification of the substance to be determined can be improved.

According to the invention in claim 3, since the conditioning potential is lower in potential than a decomposition voltage of water, increase in ion concentration in the specimen due to electrolysis of water at the time of application of the conditioning potential to the working electrode can be prevented. Therefore, a current component resulting from ionic substances resulting from electrolysis of water, which is contained in a response current measured at the time of application of the determination potential to the working electrode, can be decreased, and deterioration of accuracy in measurement of the oxidation current can be prevented.

According to the invention in claim 4, since a pulse width in application of the conditioning potential to the working electrode is from 30 to 750 milliseconds, an amount of reducing substance which experiences oxidation reaction at the time of application of the conditioning potential to the working electrode can be suppressed. Thus, variation in measured oxidation current resulting from oxidation of the reducing substance at the time of application of the determination potential to the working electrode can be suppressed.

According to the invention in claim 5, when a specimen is supplied into a cavity, a reducing substance is generated through reaction between a substance to be determined in the specimen and an enzyme. Here, before lapse of one second since sensing of supply of the specimen into the cavity, that is, before an amount of reducing substance in the specimen increases due to progress of oxidation reduction reaction between the substance to be determined and the enzyme, the conditioning potential is applied to the working electrode. Therefore, an amount of reducing substance which experiences oxidation reaction as a result of application of the conditioning potential to the working electrode can be suppressed, and an amount of reducing substance in the specimen increases due to further progress of oxidation reduction reaction between the substance to be determined and the enzyme after application of the conditioning potential to the working electrode. Thus, by application of a conditioning potential, variation in measured oxidation current resulting from oxidation of the reducing substance at the time of application of a determination potential to the working electrode can be suppressed.

According to the invention in claim 6, a reducing substance is generated through reaction between the substance to be determined in the specimen and the enzyme. Here, after lapse of at least one second since sensing of supply of the specimen into the cavity, that is, after sufficient increase in an amount of the reducing substance in the specimen due to progress of oxidation reduction reaction between the substance to be determined and the enzyme, the determination potential is applied to the working electrode. Therefore, an oxidation current resulting from oxidation of the reducing substance owing to application of the determination potential to the working electrode can reliably be measured.

According to the invention in claim 7, the cavity has a volume smaller than 0.6 µl. Thus, the substance to be determined can be quantified with the use of a small amount of specimen supplied into the cavity.

### BRIEF DESCRIPTION OF DRAWINGS

Fig. 1 is a diagram showing one example of a biosensor system employed in a substance determination method according to the present invention.
Fig. 2 is a diagram showing one example of a biosensor.
Fig. 3 is a flowchart showing one example of measurement processing.
Fig. 4 is a diagram showing one example of a potential applied to a working electrode, with a counter electrode being defined as a reference.
Fig. 5 is a diagram showing one example of a response current resulting from application of a determination potential to the working electrode.
Fig. 6 is a diagram showing a comparative example of a response current resulting from application of a determination potential to the working electrode.
Fig. 7 is a diagram showing relation between magnitude of a conditioning potential applied to the working electrode and a coefficient of variation of a measured response current.
Fig. 8 is a diagram showing relation between a pulse width of a conditioning potential applied to the working electrode and a coefficient of variation of a measured response current.
Fig. 9 is a diagram showing another example of a potential applied to the working electrode with the counter electrode being defined as the reference.
Fig. 10 is a diagram showing another example of a potential applied to the working electrode with the counter electrode being defined as the reference.
Fig. 11 is a diagram showing another example of a potential applied to the working electrode with the counter electrode being defined as the reference.
Fig. 12 is a diagram for illustrating one example of a conventional substance determination method.

### DESCRIPTION OF EMBODIMENTS

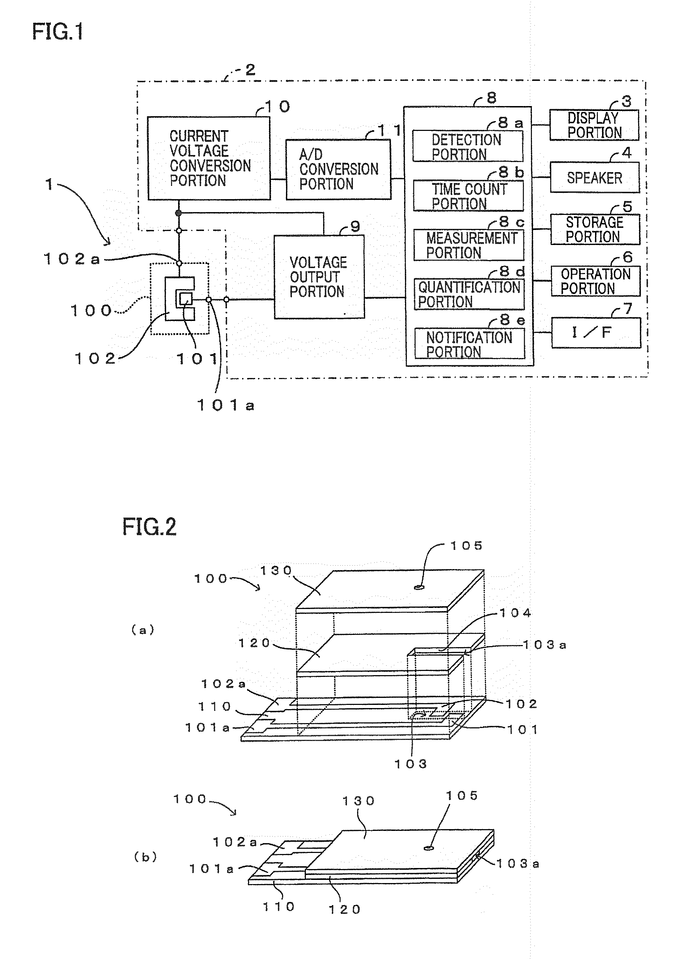

One embodiment of a substance determination method according to the present invention will be described with reference to Figs. 1 to 8.

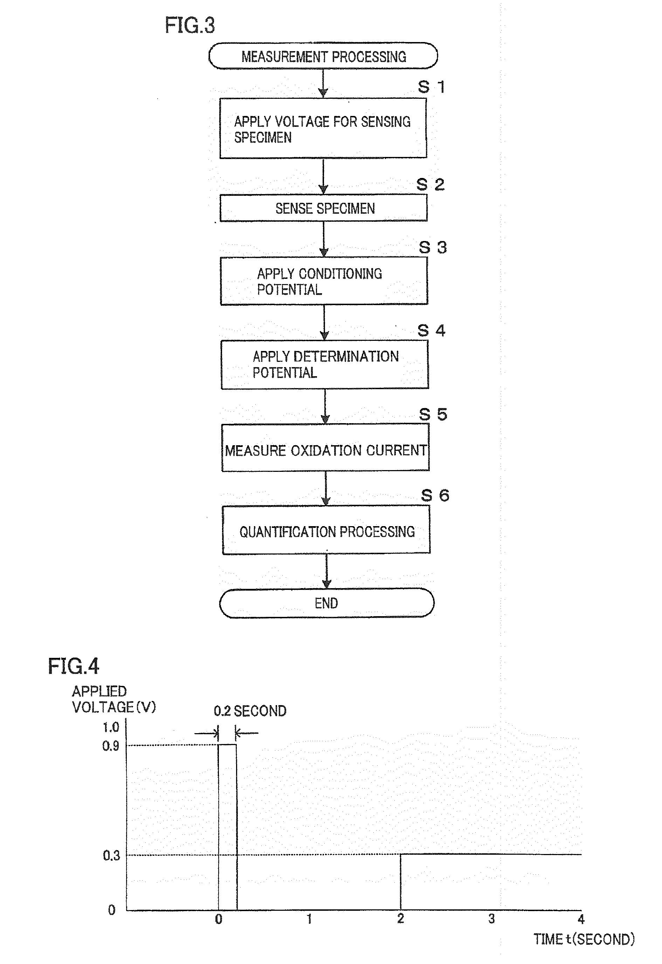

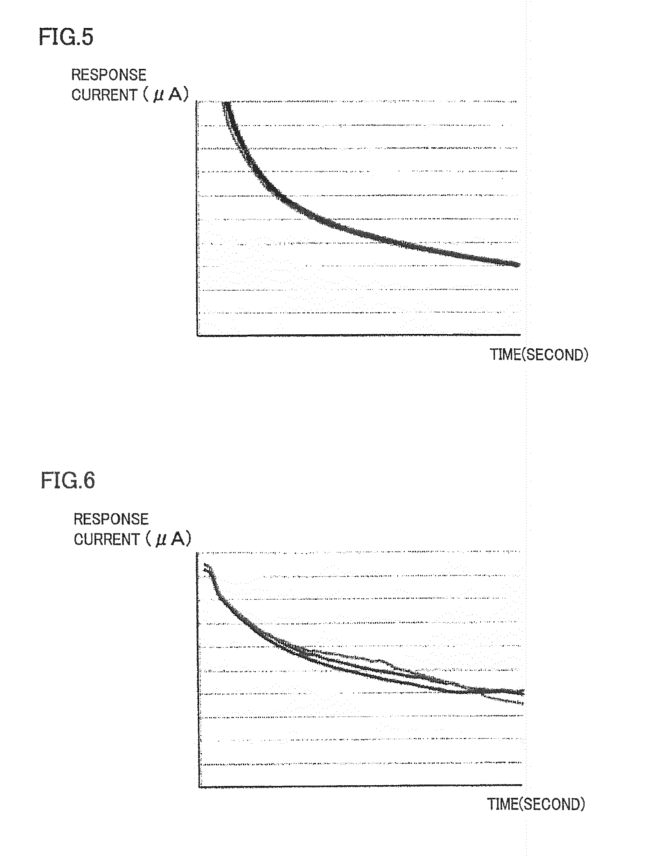

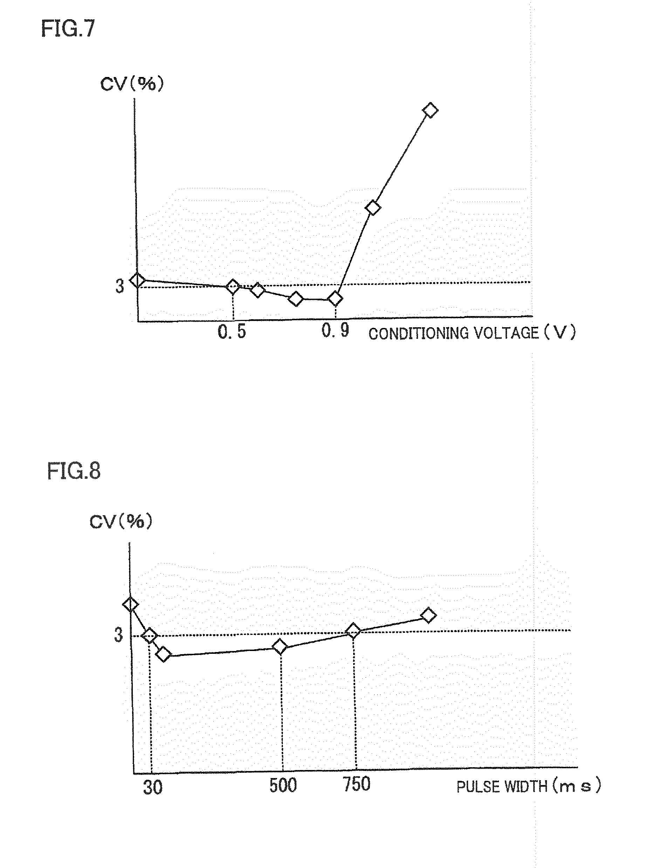

Fig. 1 is a diagram showing one example of a biosensor system employed in a substance determination method according to the present invention. Fig. 2 is a diagram showing one example of a biosensor, with (a) showing an exploded perspective view and (b) showing a perspective view. Fig. 3 is a flowchart showing one example of measurement processing. Fig. 4 is a diagram showing one example of a potential applied to a working electrode, with a counter electrode being defined as a reference. Fig. 5 is a diagram showing one example of a response current resulting from application of a determination potential to the working electrode. Fig. 6 is a diagram showing a comparative example of a response current resulting from application of a determination potential to the working electrode. Fig. 7 is a diagram showing relation between magnitude of a conditioning potential applied to the working electrode and a coefficient of variation of a measured response current. Fig. 8 is a diagram showing relation between a pulse width of a conditioning potential applied to the working electrode and a coefficient of variation of a measured response current.

### <Biosensor System>

As shown in Fig. 1, a biosensor system 1 includes a biosensor 100 having a cavity 103 into which a specimen is supplied, an electrode system including a working electrode 101 and a counter electrode 102, and a reaction layer (not shown) containing an enzyme reacting specifically with a substance to be determined and a determination instrument 2 from/to which biosensor 100 is removable/attachable. Biosensor system 1 quantifies a substance to be determined, which is contained in a specimen, by measuring an oxidation current resulting from oxidation, by application of a determination potential referenced to counter electrode 102 to working electrode 101, of a reducing substance generated through reaction with the reaction layer provided in biosensor 100, of the substance to be determined such as glucose which is contained in the specimen such as blood supplied into cavity 103 provided on a tip end side of biosensor 100 attached to determination instrument 2.

When attachment of biosensor 100 is detected, power of determination instrument 2 is automatically turned on, and as a specimen such as blood is supplied to biosensor 100, determination instrument 2 starts determination of a substance to be determined such as glucose in a specimen. Then, when quantification of a substance to be determined in the specimen is completed, a result of determination is displayed on a display portion 3 formed by such display means as an LCD, and an alarm indicating end of measurement is output from a speaker 4. The result of determination is stored in a storage portion 5 formed by such a storage medium as a memory.

Determination instrument 2 includes an operation portion 6 formed from an operation switch. As operation portion 6 is operated, various types of initial setting are made or results of past measurement stored in storage portion 5 are displayed on display portion 3.

Determination instrument 2 includes a serial interface 7 (I/F), and it can transmit and receive such data as determination results to and from an external personal computer connected through I/F 7. Storage portion 5 stores results of past determination, a conversion formula for quantifying a substance to be determined, which is contained in a specimen, based on a response current measured through application of a prescribed determination potential to working electrode 101 of biosensor 100, and a program implementing various functions as it is executed by a CPU 8.

Determination instrument 2 includes a voltage output portion 9, a current voltage conversion portion 10, and an A/D conversion portion 11. Voltage output portion 9 has a digital-analog conversion function (D/A conversion function), and based on a control command from CPU 8, it outputs a constant reference potential to counter electrode 102 of biosensor 100 attached to determination instrument 2, and outputs a prescribed potential referenced to a reference potential applied to counter electrode 102 to working electrode 101.

Current voltage conversion portion 10 has a common current voltage conversion circuit formed from an operational amplifier or a resistor, and it converts into a voltage signal, a response current which flows between working electrode 101 and counter electrode 102 as a prescribed determination potential is applied to working electrode 101 of biosensor 100 from voltage output portion 9, such that the signal can be taken into CPU 8. A/D conversion portion 11 converts the voltage signal converted by current voltage conversion portion 10 into a digital signal. Then, the digital signal converted by A/D conversion portion 11 is taken into CPU 8 and subjected to prescribed operation in CPU 8. Thus, the digital signal is converted from a voltage signal into a current signal.

CPU 8 has functions below, by executing various programs stored in storage portion 5 for quantifying a substance to be determined which is contained in the specimen.

A detection portion 8a detects change in resistance value due to short-circuiting between working electrode 101 and counter electrode 102 which is caused by a specimen composed of a liquid and thereby detects supply of the specimen into cavity 103 provided in biosensor 100, by monitoring a value for a current which flows between working electrode 101 and counter electrode 102, which is input to CPU 8 through A/D conversion portion 11. A time count portion 8b counts, for example, a time period which has elapsed since detection of supply of a specimen into cavity 103 by detection portion 8a or a time period of application of a prescribed potential to working electrode 101 from voltage output portion 9, based on a clock signal output from a not-shown clock circuit.

A measurement portion 8c measures a response current which flows between working electrode 101 and counter electrode 102 at the time when a prescribed determination potential referenced to counter electrode 102 is applied to working electrode 101 from voltage output portion 9.

A quantification portion 8d quantifies a substance to be determined, based on a response current measured by measurement portion 8c. Specifically, relation between a response current measured at the time when a prescribed determination voltage referenced to counter electrode 102 is applied to working electrode 101 and a concentration of the substance to be determined which is contained in the specimen is measured in advance, so that a conversion formula for calculating by conversion a concentration of the substance to be determined from a response current is derived and stored in advance in storage portion 5. Then, a substance to be determined is quantified based on the conversion formula stored in storage portion 5 and the actually measured response current.

A notification portion 8e gives notification by displaying a result of quantification by quantification portion 8d on display portion 3 or outputting an alarm indicating end of determination from speaker 4.

### <Biosensor>

As shown in Fig. 2, biosensor 100 is formed in such a manner that an electrode layer 110 formed of an insulating material such as ceramics, glass, plastic, paper, a biodegradable material, or polyethylene terephthalate and provided with working electrode 101 and counter electrode 102, a cover layer 130 having an air vent 105 formed, and a spacer layer 120 having a slit 104 for forming cavity 103 formed and arranged between electrode layer 110 and cover layer 130 are stacked and bonded with their tip end sides being aligned as shown in Fig. 2 (a). Then, biosensor 100 is attached to determination instrument 2 as it is introduced and attached through a prescribed port of determination instrument 2 from a rear end side.

In this embodiment, electrode layer 110 is formed from a substrate composed of polyethylene terephthalate. As a pattern is formed through laser processing or photolithography in an electrode film composed of such a noble metal as platinum, gold, or palladium or of such a conductive substance as carbon, copper, aluminum, titanium, ITO, or ZnO and formed on the substrate of electrode layer 110 through screen printing or sputtering vapor deposition, working electrode 101 and counter electrode 102 as well as electrode patterns 101a and 102a electrically connecting working electrode 101 and counter electrode 102 to determination instrument 2 are provided.

Spacer layer 120 is formed from a substrate composed of polyethylene terephthalate. Slit 104 for forming cavity 103 is formed substantially in a center of a tip edge portion of the substrate. Spacer layer 120 is stacked on and bonded to electrode layer 110 such that their tip ends are aligned as shown in Fig. 2 (a).

The reaction layer is formed by dropping, before cover layer 130 is stacked, a reagent containing a thickening agent such as carboxymethylcellulose or gelatin, an enzyme, a mediator, or an additive such as amino acid or an organic acid, onto working electrode 101 and counter electrode 102 formed as spacer layer 120 is stacked on electrode layer 110 and partially exposed at cavity 103. In order to smoothly supply a specimen such as blood to cavity 103, a hydrophilizing agent such as a surfactant or phospholipid is applied to an inner wall of cavity 103.

Glucose oxidase, lactate oxidase, cholesterol oxidase, alcohol oxidase, sarcosine oxidase, fructosyl amine oxidase, pyruvic oxidase, glucose dehydrogenase, lactate dehydrogenase, alcohol dehydrogenase, hydroxybutyrate dehydrogenase, cholesterol esterase, creatininase, creatinase, or DNA polymerase can be employed as an enzyme, and various sensors can be formed by making selection in accordance with a substance to be determined, an enzyme for which is desirably detected.

For example, with glucose oxidase or glucose dehydrogenase, a glucose sensor for detecting glucose in a specimen can be formed, with alcohol oxidase or alcohol dehydrogenase, an alcohol sensor for detecting ethanol in a specimen can be formed, with lactate oxidase, a lactate sensor for detecting lactic acid in a specimen can be formed, and with a mixture of cholesterol esterase and cholesterol oxidase, a total cholesterol sensor can be formed.

Potassium ferricyanide, ferrocene, a ferrocene derivative, benzoquinone, a quinone derivative, an osmium complex, or a ruthenium complex can be employed as a mediator.

Carboxymethylcellulose, carboxyethylcellulose, polyethyleneimine, DEAE cellulose, dimethylaminoethyl dextran, carageenan, sodium alginate, or dextran can be employed as a thickening agent. A surfactant such as Triton X100, Tween 20, or sodium bis(2-ethylhexyl)sulfosuccinate, or phosphoslipid such as lecithin can be employed as a hydrophilizing agent. In order to lessen variation in concentration of ions contained in a specimen such as blood, a buffer such as phosphoric acid may be provided.

Cover layer 130 is formed from a substrate composed of polyethylene terephthalate, and in the substrate, air vent 105 communicating with cavity 103 at the time when it is stacked on spacer layer 120 is formed. After the reaction layer is formed on working electrode 101 and counter electrode 102 exposed at cavity 103, cover layer 130 is stacked on and bonded to spacer layer 120. Thus, biosensor 100 having a specimen introduction port 103a communicating with cavity 103 formed at a tip end for supply of a specimen such as blood into cavity 103 is formed. Slit 104 is formed in spacer layer 120 such that cavity 103 of biosensor 103 has a volume smaller than approximately 0.6 µl.

In this embodiment, biosensor system 1 is formed for the purpose of quantification of glucose in blood. The reaction layer containing glucose dehydrogenase as an enzyme reacting specifically with glucose representing a substance to be determined and potassium ferricyanide as a mediator, which will be a reducing substance as a result of reduction by electrons generated through reaction between glucose representing a substance to be determined and glucose dehydrogenase, is provided on working electrode 101 and counter electrode 102 exposed at cavity 103.

In biosensor 100 thus constructed, by bringing a specimen in contact with specimen introduction port 103a at the tip end, the specimen is attracted toward air vent 105 owing to a capillary phenomenon, so that the specimen is supplied into cavity 103. Then, as the reaction layer is dissolved in the specimen supplied into cavity 103, electrons are emitted through enzyme reaction between glucose representing a substance to be determined in the specimen and glucose dehydrogenase, emitted electrons reduce ferricyanide ions, and ferrocyanide ions representing a reducing substance are generated.

As the specimen is supplied into cavity 103, determination instrument 2 removes such an impurity as soil or dust adhering to working electrode 101 and counter electrode 102 by applying to working electrode 101 at least once, a pulse of conditioning potential higher than a determination potential with counter electrode 102 being defined as the reference before application of the determination potential for measurement of an oxidation current. Determination instrument 2 quantifies glucose in the specimen by measuring an oxidation current which flows between working electrode 101 and counter electrode 102 by electrochemically oxidizing a reducing substance through application of a determination potential equal to or higher than an oxidation potential at which a reducing substance generated through oxidation reduction reaction resulting from solution of the reaction layer into the specimen is oxidized to working electrode 101 of biosensor 100 with counter electrode 102 being defined as the reference, after application of the conditioning potential to working electrode 101.

Though biosensor 100 is formed to have a dual-electrode structure having working electrode 101 and counter electrode 102 in this embodiment, biosensor 100 may be formed to have a triple-electrode structure by further providing a reference electrode. In this case, while counter electrode 102 is grounded and a reference potential is applied to the reference electrode from voltage output portion 9, a prescribed determination potential referenced to counter electrode 102 should only be applied to working electrode 101.

Though supply of a specimen into cavity 103 is detected by monitoring a current which flows between working electrode 101 and counter electrode 102 as a result of application of a prescribed voltage across working electrode 101 and counter electrode 102 in this embodiment, supply of the specimen into cavity 103 may be detected by further providing an electrode for sensing a specimen and monitoring a current which flows between counter electrode 102 and the electrode for sensing the specimen as a result of application of a prescribed voltage across counter electrode 102 and the electrode for sensing the specimen. At least cover layer 130 among electrode layer 110, spacer layer 120, and cover layer 130 forming biosensor 100 is desirably formed from a transparent member such that supply of the specimen into cavity 103 can visually be recognized.

### <Measurement Processing>

One example of measurement processing performed in biosensor system 1 will now be described. As a not-shown detection circuit detects attachment of biosensor 100 to determination instrument 2, a voltage for sensing a specimen for detecting supply of a specimen composed of blood into cavity 103 in biosensor 100 is applied across working electrode 101 and counter electrode 102 (step S1). Then, as the specimen is supplied into cavity 103 and liquid junction between working electrode 101 and counter electrode 102 by the specimen is established, a current which flows between working electrode 101 and counter electrode 102 increases and hence change in resistance value is sensed. Thus, detection portion 8a detects supply of the specimen into cavity 103 (step S2).

When detection portion 8a detects supply of the specimen into cavity 103, voltage output portion 9 applies a conditioning potential referenced to counter electrode 102 to working electrode 101 before 1 second or desirably 0.5 second elapses since sensing of supply of the specimen into cavity 103 (step S3). In this embodiment, as shown in Fig. 4, from time t=0 at which detection portion 8a detects supply of the specimen into cavity 103, a conditioning potential of approximately 0.9 V referenced to counter electrode 102 with a pulse width of approximately 0.2 second is applied to working electrode 101.

Then, after the conditioning potential is applied to working electrode 101 and after at least one second has elapsed since sensing of supply of the specimen into cavity 103, voltage output portion 9 applies a determination potential referenced to counter electrode 102 to working electrode 101 (step S4). In this embodiment, from time t=2 at which 2 seconds have elapsed since detection by detection portion 8a of supply of the specimen into cavity 3, a determination potential of approximately 0.3 V referenced to counter electrode 102 is applied to working electrode 101.

In succession, after the determination potential is applied to working electrode 101, measurement portion 8c measures a response current (an oxidation current) approximately 3 to 5 seconds after sensing of supply of the specimen into cavity 103 (step S5). Then, glucose contained in the specimen is quantified based on the measured current value of the response current and the conversion formula stored in storage portion 5, and notification portion 8e gives notification of a result of determination. Thus, the processing ends (step S6).

In this embodiment, a determination potential is set to be not lower than an oxidation potential at which ferrocyanide ions representing a reducing substance resulting from enzyme reaction of glucose are oxidized, and it is set to approximately 0.3 V. A conditioning potential is set to approximately 0.9 V, which is higher than a determination potential and lower in potential than a decomposition voltage of water (approximately 1 V).

### <Comparison of Response Current>

Fig. 5 shows results of measurement of a response current three times under the same conditions as in the "measurement processing" described above. Fig. 6 shows results of measurement of a response current three times under the same conditions as in the "measurement processing" described above, except for not applying a conditioning potential to working electrode 101.

As shown in Fig. 5, when a conditioning potential is applied to working electrode 101 before a determination potential is applied to working electrode 101 as in the "measurement processing" described above, a substantially similar response current is measured in a stable manner. On the other hand, when no conditioning potential is applied to working electrode 101 before a determination potential is applied to working electrode 101, a measured response current is unstable.

As above, according to this embodiment, after a specimen is supplied into cavity 103 in biosensor 100 and before a determination potential for measuring an oxidation current resulting from oxidation of a reducing substance generated through reaction between a substance to be determined in the specimen and an enzyme is applied to working electrode 101, a pulse of conditioning potential higher than a determination potential with counter electrode 102 being defined as the reference is applied at least once to working electrode 101. Therefore, an impurity adhering to working electrode 101 and counter electrode 102 which will electrochemically react at the time of application of a determination potential to working electrode 101 electrochemically reacts as a result of application of a conditioning potential to working electrode 101 with counter electrode 102 being defined as the reference, and thus it is removed from working electrode 101 and counter electrode 102.

Therefore, influence by a current component different from an oxidation current resulting from oxidation of a reducing substance generated through reaction between the substance to be determined in the specimen and the enzyme, of current components contained in a response current resulting from application of a determination potential referenced to counter electrode 102 to working electrode 101, can be lessened. Thus, a response current can be measured in a stable manner and determination accuracy in quantification of the substance to be determined which is contained in the specimen can be improved.

Since the determination potential is equal to or higher than the oxidation potential at which the reducing substance resulting from enzyme reaction of the substance to be determined is oxidized, variation in concentration of the reducing substance in the specimen can be suppressed without increase in an amount of the reducing substance contained in the specimen due to reduction reaction caused by application of the determination potential to working electrode 101, and an oxidation current resulting from oxidation of the reducing substance can be stabilized. Therefore, determination accuracy in quantification of the substance to be determined can be improved.

Since the conditioning potential is lower in potential than a decomposition voltage of water, increase in ion concentration in the specimen due to electrolysis of water at the time of application of the conditioning potential to working electrode 101 can be prevented. Therefore, a current component resulting from ionic substances resulting from electrolysis of water, which is contained in the response current measured at the time of application of the determination potential to working electrode 101, can be decreased, and deterioration of accuracy in measurement of the oxidation current can be prevented.

Relation between magnitude of a conditioning potential applied to working electrode 101 and a coefficient of variation (CV) of a response current (an oxidation current) measured at the time of application of a determination potential to working electrode 101 was measured, and a result as follows was obtained. Namely, as shown in Fig. 7, when a conditioning potential is from approximately 0.5 V to approximately 0.9 V which is higher than an oxidation potential and lower than a decomposition voltage of water, a CV of a response current measured at the time of application of the determination potential to working electrode 101 can be suppressed to 3% or lower.

Since a pulse width in application of a conditioning potential to working electrode 101 is set to 0.2 second, an amount of reducing substance which experiences oxidation reaction at the time of application of the conditioning potential to working electrode 101 can be suppressed. Therefore, variation in measured oxidation current resulting from oxidation of a reducing substance at the time of application of a determination potential to working electrode 101 can be suppressed.

A pulse width of a pulse of conditioning potential applied to working electrode 101 is not limited to 0.2 second. Relation between a pulse width of a conditioning potential applied to working electrode 101 and a coefficient of variation (CV) of a response current (an oxidation current) measured at the time of application of a determination potential to working electrode 101 was measured, and a result as follows was obtained. Namely, as shown in Fig. 8, when a pulse width of a conditioning potential is set to approximately 30 milliseconds to approximately 750 milliseconds, a CV of a response current measured at the time of application of a determination potential to working electrode 101 can be suppressed to 3% or lower.

When a specimen is supplied into cavity 103, a reducing substance is generated through reaction between a substance to be determined in the specimen and an enzyme. Here, before lapse of one second since sensing of supply of the specimen into cavity 103, that is, before an amount of reducing substance in the specimen increases due to progress of oxidation reduction reaction between the substance to be determined and the enzyme, a conditioning potential is applied to working electrode 101. Therefore, an amount of reducing substance which experiences oxidation reaction due to application of the conditioning potential to working electrode 101 can be suppressed, and an amount of reducing substance in the specimen increases due to further progress of oxidation reduction reaction between the substance to be determined and the enzyme after application of the conditioning potential to working electrode 101. Thus, by application of a conditioning potential, variation in measured oxidation current resulting from oxidation of the reducing substance at the time of application of a determination potential to working electrode 101 can be suppressed.

A reducing substance is generated through reaction between the substance to be determined in the specimen and the enzyme. Here, after lapse of at least one second since sensing of supply of the specimen into cavity 103, that is, after sufficient increase in an amount of the reducing substance in the specimen due to progress of the oxidation reduction reaction between the substance to be determined and the enzyme, the determination potential is applied to working electrode 101. Therefore, an oxidation current resulting from oxidation of the reducing substance owing to application of the determination potential to working electrode 101 can be measured reliably in a stable manner.

Even though a cavity has a volume smaller than 0.6 µl and a specimen supplied into cavity 103 is small in amount, oxidation of a reducing substance resulting from oxidation reduction reaction between a substance to be determined and an enzyme is prevented by application of a conditioning potential higher than an oxidation potential of the reducing substance before lapse of one second since sensing of supply of the specimen into cavity 103 as described above. Therefore, a substance to be determined can be quantified with the use of a small amount of specimen.

The present invention is not limited to the embodiment described above, and various modifications other than the above can be made without departing from the spirit thereof. For example, an ethanol sensor or a lactate sensor may be formed by changing combination with an enzyme and a mediator to be contained in the reaction layer of biosensor 100 described above. The reaction layer does not necessarily have to contain a mediator. In this case, an oxidation current resulting from oxidation of a reducing substance such as hydrogen peroxide or a reductant of an enzyme produced through enzyme reaction of a substance to be determined such as glucose should only be measured.

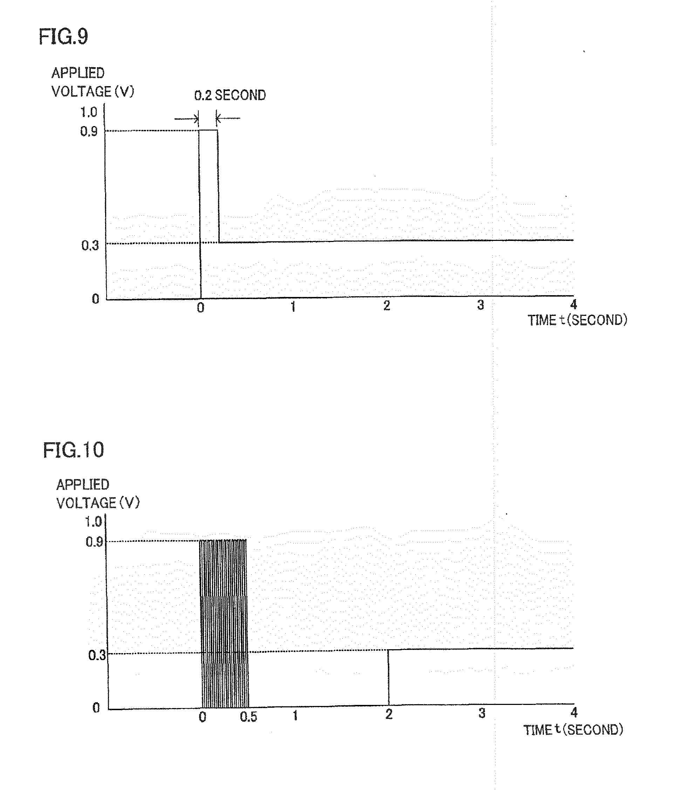

A determination potential may be applied to working electrode 101 before lapse of at least one second since sensing of supply of a specimen into cavity 103. For example, as shown in Fig. 9, voltage output portion 9 may apply a determination potential of approximately 0.3 V to working electrode 101 immediately after application of a conditioning potential of approximately 0.9 V referenced to counter electrode 102 to working electrode 101 with a pulse width of approximately 0.2 second.

A pulse of conditioning potential may be applied to working electrode 101 a plurality of times after sensing of supply of a specimen into cavity 103. For example, as shown in Fig. 10, before 1 second or desirably 0.5 second elapses since sensing of supply of a specimen into cavity 103, voltage output portion 9 may apply to working electrode 101 a plurality of times, a pulse of conditioning potential of 0.9 V referenced to counter electrode 102.

A conditioning potential does not have to be constant in potential. For example, as shown in Fig. 11, before 1 second or desirably 0.5 second elapses since sensing of supply of a specimen into cavity 103, voltage output portion 9 may apply to working electrode 101 a plurality of times, a pulse of conditioning potential of 0.9 V referenced to counter electrode 102, which is different in potential. Figs. 9 to 11 are diagrams showing other examples of a potential applied to the working electrode with the counter electrode being defined as the reference.

A conditioning potential does not necessarily have to be applied to working electrode 101 immediately after sensing of supply of a specimen into cavity 103. A conditioning potential may be applied to working electrode 101 more than 1 second after sensing of supply of a specimen into cavity 103. A pulse of conditioning potential different in pulse width and potential may be applied to working electrode 101 a plurality of times. A conditioning potential not lower than a decomposition voltage of water may be applied to working electrode 101.

Though a voltage across working electrode 101 and counter electrode 102 is set to 0 V after application of a conditioning potential referenced to counter electrode 102 to working electrode 101 in the embodiment described above, a circuit may be opened after application of the conditioning potential to working electrode 101, in order to promote oxidation reduction reaction between a substance to be determined and an enzyme.

Cavity 103 in biosensor 100 is desirably formed to have a smaller volume.

### INDUSTRIAL APPLICABILITY

The present invention can be applied to a substance determination method with the use of various biosensors.

### REFERENCE SIGNS LIST

100 biosensor; 101 working electrode; 102 counter electrode; and 103 cavity.

## Claims

1. A substance determination method for quantification of a substance to be determined, by measuring, by using a biosensor having a cavity into which a specimen is supplied; an electrode system including a working electrode and a counter electrode; and a reaction layer containing an enzyme reacting specifically with a substance to be determined, an oxidation current resulting from oxidation, through application of a determination potential referenced to said counter electrode to said working electrode, of a reducing substance generated through reaction between said substance to be determined which is contained in the specimen supplied into said cavity and said reaction layer, comprising:
applying to said working electrode at least once, a pulse of conditioning potential higher than said determination potential with said counter electrode being defined as a reference after said specimen is supplied into said cavity and before said determination potential is applied to said working electrode.

2. The substance determination method according to claim 1, wherein
said determination potential is equal to or higher than an oxidation potential at which said reducing substance is oxidized.

3. The substance determination method according to claim 1 or 2, wherein said conditioning potential is lower in potential than a decomposition voltage of water.

4. The substance determination method according to any of claims 1 to 3, wherein
a pulse width in application of said conditioning potential to said working electrode is from 30 to 750 milliseconds.

5. The substance determination method according to any of claims 1 to 4, wherein
said conditioning potential is applied before lapse of one second since sensing of supply of said specimen into said cavity.

6. The substance determination method according to any of claims 1 to 5, wherein
said determination potential is applied after lapse of at least one second since sensing of supply of said specimen into said cavity.

7. The substance determination method according to any of claims 1 to 6, wherein
said cavity has a volume smaller than 0.6 µl.
